# EUROPÄISCHE PATENTSCHRIFT

(11) **EP 2 019 821 B1**
(45) Veröffentlichungstag und Bekanntmachung des Hinweises auf die Patenterteilung: **09.12.2009**
(21) Anmeldenummer: 07728960.1
(22) Anmeldetag: 10.05.2007
(51) Int. Cl.: C07D 207/26

(54) **VERFAHREN ZUR KONTINUIERLICHEN HERSTELLUNG VON N-ETHYL-2-PYRROLIDON (NEP)**
PROCESS FOR CONTINUOUSLY PREPARING N- ETHYL-2-PYRROLIDONE (NEP)
PROCÉDÉ DE FABRICATION EN CONTINU DE N-ÉTHYL-2-PYRROLIDONE (NEP)

(30) Priorität: 16.05.2006 EP 06114031
(43) Veröffentlichungstag der Anmeldung: 04.02.2009
(73) Patentinhaber: BASF SE, 67056 Ludwigshafen (DE)
(72) Erfinder: SCHMIDTKE, Helmut, 64625 Bensheim (DE); VERSCH, Ralph, 67346 Speyer (DE); SIMON, Silke, Wilmslow SK9 1LL (GB); OTT, Karl, 68723 Plankstadt (DE); DREWS, Ronald, 69488 Birkenau (DE); MOLLNER, Stephanie, 68163 Mannheim (DE)
(86) Internationale Anmeldenummer: PCT/EP2007/054508
(87) Internationale Veröffentlichungsnummer: WO 2007/131929

(56) Entgegenhaltungen:
- WO-A-03/053924
- WO-A-2005/121083
- DATABASE CAPLUS [Online] CHEMICAL ABSTRACTS SERVICE, COLUMBUS, OHIO, US; QIU, G.: "Preparation of 1-ethyl-2-aminomethyltetrahydropyrrole and its intermediate N-ethyl-2-pyrrolidone" XP002447193 gefunden im STN Database accession no. 2000:230205 -& CN 1 199 730 A (QIU GUOXIANG [CN]) 25. November 1998 (1998-11-25)
- DATABASE CAPLUS [Online] CHEMICAL ABSTRACTS SERVICE, COLUMBUS, OHIO, US; XU, ZH. ET AL: "Production method of N-ethyl-.alpha.-pyrrolidone" XP002447194 gefunden im STN Database accession no. 2000:597002 -& DATABASE EPODOC EUROPEAN PATENT OFFICE, THE HAGUE, NL; XU, ZH. ET AL: "Production method of N-ethyl-.alpha.-pyrrolidone" XP002447220 -& CN 1 237 578 A (HEFEI JIANGHUAI CHEMICAL FERTI [CN]) 8. Dezember 1999 (1999-12-08)

## Beschreibung

Die vorliegende Erfindung betrifft ein Verfahren zur kontinuierlichen Herstellung von N-Ethyl-2-pyrrolidon (NEP) durch Umsetzung von gamma-Butyrolacton mit Monoethylamin in der Flüssigphase.

NEP ist wegen bestimmter vorteilhafter Eigenschaften ein wichtiges, vielseitiges Lösungsmittel und Reaktionsmedium für die chemische Industrie.
Aufgrund seiner u.a. günstigen toxikologischen Eigenschaften kann es vorteilhaft andere Lösungsmittel substituieren; vgl. WO-A-05/090447 (BASF AG).
WO 2005/121083 beschreibt ein Verfahren zur Synthese von N-Alkyl-pyrrolidonen aus Monoalkylaminen und gamma-Butyrolacton in der flüssigen Phase.
JP-B-74 020 585 (Mitsubishi Chem. Ind. Ltd.) beschreibt die Herstellung von Pyrrolidonen aus GBL und primären Alkylaminen in Gegenwart von Wasser bei 200 bis 300°C.

JP-B-47 021 420 (Mitsubishi Chemical Inds) betrifft ebenfalls die Herstellung von Pyrrolidonen aus GBL und primären Alkylaminen in Gegenwart von Wasser.

JP-A-2001 002638 (Tonen Kagaku KK) offenbart eine Herstellung von reinen Pyrrolidonen durch Anpassung der Verweilzeit und Temperatur der Flüssigkeit im unteren Teil einer Destillationskolonne.

JP-A-2001 354646 (Mitsubishi Chem. Corp.) beschreibt eine Pyrrolidon-Zusammensetzung hergestellt aus GBL und NH₃, primärem Amin, sekundärem Amin und/oder tertiärem Amin.

US 4,885,371 (GAF Chemicals Corp.) betrifft die Herstellung von N-Alkyl-lactamen aus entsprechenden Lactonen und einem Alkylamin in Gegenwart katalytischer Mengen an Borhydriden.

EP-A-1 004 577 (Mitsubishi Chem. Corp.) lehrt die Herstellung von N-Alkyl-2-pyrrolidonen durch Umsetzung von GBL oder 4-Hydroxybuttersäure mit einem Alkylamin-Gemisch, wobei im Alkylamin-Gemisch der Gehalt an primärem Amin ≤ 85 Gew.-% beträgt.

EP-A-1 201 652 (Dairen Chem. Corp.) beschreibt ein Verfahren zur Herstellung von Lactamen aus Lactonen in Gegenwart von kristallinen Alumosilikat-Zeolith-Katalysatoren und Wasser.

Erfindungsgemäß wurde erkannt, dass sich aus dem Stand der Technik unter anderem folgende Nachteile ergeben:
a) niedrige Raum-Zeit-Ausbeuten an NEP,
b) der Einsatz von größeren Mengen Wasser in der Synthese, der u.a. zu niedrigen Raum-Zeit-Ausbeuten an NEP und hohen Energiekosten für die Vorheizung des Eduktes und die Aufbereitung des Reaktionsaustrages führt, und
c) der große molare Überschuss an Monoethylamin bezogen auf GBL, der einen hohen technischen Aufwand bei der Aufarbeitung des Reaktiönsaustrags und Rückgewinnung des unumgesetzten Monoethylamins und damit hohe Investitions- und Energiekosten bedingt.

Der vorliegenden Erfindung lag die Aufgabe zugrunde, unter Überwindung von einem oder mehreren Nachteilen des Stands der Technik, ein verbessertes, selektives Verfahren zur Herstellung von NEP in hohen Ausbeuten und Raum-Zeit-Ausbeuten (RZA) und hoher Qualität (z.B. Reinheit > 99,5 Gew.%, APHA-Farbzahl ≤ 20, GBL-Restgehalt < 0,05 Gew.-%) aufzufinden.

Demgemäß wurde ein Verfahren zur kontinuierlichen Herstellung von N-Ethyl-2-pyrrolidon (NEP) durch Umsetzung von gamma-Butyrolacton (GBL) mit Monoethylamin (MEA) in der Flüssigphase gefunden, welches dadurch gekennzeichnet ist, dass man GBL und MEA in einem Molverhältnis im Bereich von 1 : 1,08 bis 1 : 2 einsetzt und die Umsetzung bei einer Temperatur im Bereich von 320 bis 420°C und einem Absolutdruck im Bereich von 70 bis 120 bar durchführt.

Vorzugsweise wird die erfindungsgemäße Umsetzung bei einer Temperatur im Bereich von 330 bis 410°C, insbesondere bei einer Temperatur im Bereich von 340 bis 380°C, ganz besonders bei einer Temperatur im Bereich von 345 bis 370°C, z.B. im Bereich von 350 bis 366°C, durchgeführt.

Die erfindungsgemäße Umsetzung wird vorzugsweise bei einem Absolutdruck im Bereich von 70 bis 110 bar, insbesondere bei einem Absolutdruck im Bereich von 80 bis 110 bar, ganz besonders bei einem Absolutdruck im Bereich von 80 bis 105 bar, z.B. im Bereich von 90 bis 100 bar, durchgeführt.

Das Molverhältnis der Einsatzstoffe GBL : Monoethylamin beträgt im erfindungsgemäßen Verfahren bevorzugt 1 : (1,01 bis 2,0), weiter bevorzugt 1 : (1,02 bis 1,5), insbesondere 1 : (1,03 bis 1,3), ganz besonders 1 : (1,04 bis 1,2), ganz besonders bevorzugt 1 : (1,04 bis 1,15), z.B. 1 : 1,14.

Das Einsatzstoffgemisch enthält am Reaktoreingang bevorzugt weniger als 40 Gew.-%, z.B. weniger als 15 Gew.%, besonders weniger als 10 Gew.-%, ganz besonders weniger als 5 Gew.-%, insbesondere weniger als 1 Gew.-%, z.B. 0 bis 0,9 Gew.-%, Wasser. In einer besonders bevorzugten Ausführungsform enthält das Einsatzstoffgemisch kein Wasser.

### Das erfindungsgemäße Verfahren kann z.B. wie folgt ausgeführt werden:

Als Reaktor dient vorzugsweise ein aufrecht stehendes, schlankes Hochdruckrohr, das bevorzugt nach einem Wärmetauscher mit Umgang mit einem über einen Druckregler gesteuerten Entspannungsventil ausgestattet ist. Die Feedströme werden vorzugsweise vom Reaktoraustrag vorgeheizt, so dass im Reaktor durch die Exothermie die erfindungsgemäßen Reaktionstemperaturen eingestellt werden können. Im Reaktor sind bevorzugt mehrere Siebböden zur Verhinderung einer Rückvermischung und besseren Ausbildung einer Pfropfenströmung (quasi Rührkesselkaskade) eingebaut. Die Anzahl der Siebböden kann z.B. 10 bis 40, bevorzugt 20 bis 30, betragen.

Das MEA wird bevorzugt in wässriger Lösung eingesetzt, z.B. als 60 bits 80 Gew.-%ige Lösung, besonders als ≤ 40 Gew.-%ige, insbesondere ≤ 31 Gew.-%ige, ganz besonders ≤ 28,5 Gew.%ige, Lösung, wobei bevorzugt das Wasser vor dem Eintrag in den Reaktor, bevorzugt destillativ, abgetrennt wird.

Das MEA wird bevorzugt in einer Reinheit (berechnet wasserfrei) von ≥ 90 Gew.-%, insbesondere ≥ 98 Gew.%, ganz besonderes ≥ 99,8 Gew.-%, und eingesetzt.

Bevorzugt flüssiges Monoethylamin, gegebenenfalls gemischt mit zurückgewonnenem Monoethylamin aus der Aufarbeitung (siehe unten), wird mit einer Pumpe, z.B. Membranpumpe, bevorzugt über einen rekuperativen Wärmetauscher W und bevorzugt über einen z.B. dampfbetriebenen Aufheizer an das untere Ende des Reaktors, bevorzugt Rohrreaktors, gepumpt (bevorzugte Sumpffahrweise).

GBL wird mit einer Pumpe, z.B. Membranpumpe, bevorzugt über einen z.B. dampfbetriebenen Aufheizer ebenfalls an das untere Ende des Reaktors, bevorzugt Rohrreaktors, geführt, wobei eine Durchmischung der Einsatzstoffe stattfindet und die Einsatzstoffe GBL und Monoethylamin im oben angegebenen, erfindungsgemäßen Molverhältnis vorliegen.

Das flüssige Monoethylamin und das GBL werden in einer besonders bevorzugten Ausführungsform dem Rohrreaktor im Zentrum des Reaktorbodens durch einen Zweistoff-Injektor separat zugeführt. Während das GBL hierbei mit hoher Strömungsgeschwindigkeit (bevorzugt 4-12 m/s) durch die Zentraldüse eingeleitet wird, gelangt das flüssige Monoethylamin an der Außenseite dieser Düse durch einen Ringspalt in den Reaktor. Die Edukte werden hierbei als Treibstrahl in ein Umlaufrohr geleitet, das sich im Eintrittsbereich des Reaktors befindet, ca. ein Drittel des freien Rohrquerschnittes umschließt und eine Länge von bevorzugt 1,5 bis 2,0 m besitzt. Die Rezirkulation von Flüssigkeit, die an der Außenseite des Umlaufrohres erfolgt und durch den Treibstrahl in Gang gehalten wird, führt zu einem intensiven ersten Kontakt der Reaktionspartner im oben angegebenen, erfindungsgemäßen Molverhältnis.

Im Reaktor erfolgt unter den oben angegebenen Temperaturen und Drücken die kontinuierliche Umsetzung des GBLs mit Monoethylamin in flüssiger Phase in exothermer. Reaktion zu NEP.

Die Umsetzung wird bevorzugt in Abwesenheit eines Katalysators durchgeführt.

Die Reaktorbelastung liegt bevorzugt im Bereich von 0,5 bis 2 kg GBL / (I_{Reaktor} • h), besonders 1,0 bis 1,5 kg GBL / (I_{Reaktor} • h).

Die mittleren Verweilzeiten des Reaktionsgemischs im Reaktor bzw. in allen Reaktoren, wenn mehrere eingesetzt werden (vgl. weiter unten), ermittelt mit der Dichte von GBL und Monoethylamin (flüssig) bei Normaltemperatur (20°C), betragen je nach Belastung bevorzugt 10 bis 60 Min., bevorzugt 15 bis 30 Min.

Die Raum-Zeit-Ausbeuten an NEP im Reaktorrohaustrag betragen bevorzugt ≥ 0,5 kg NEP / (h • I_{Reaktor}), besonders 1 kg NEP / (h • I_{Reaktor}), weiter besonders 1 bis 3 kg NEP / (h • I_{Reaktor}), ganz besonders 1,1 bis 2,5 kg NEP / (h • I_{Reakt}), z.B. 2 kg NEP / (h • I_{Reaktor}).

(I_{Reaktor} = Reaktorvolumen in Liter, bei mehreren Reaktoren Gesamtreaktorvolumen in Liter).

Das erhaltene Reaktionsprodukt wird aus dem Reaktor bevorzugt kontinuierlich als Zulauf in eine Destillationskolonne K1 entspannt, wobei bevorzugt der Reaktoraustrag zunächst zur Aufheizung des Ethylamins ganz oder teilweise über den rekuperativen Wärmetauscher W (siehe oben) geleitet und dabei abgekühlt wird.

Dem Zulauf der Kolonne K1 kann zuvor Kopfdestillat der Destillationskolonne K2 (Hauptbestandteil: NEP. Siehe unten) zur Rückgewinnung des im Kopfdestillat der K2 enthaltenen NEPs zugemischt werden.

In der Destillationskolonne K1 werden, z.B. bei 40 bis 240°C und 1 bis 2 bar, noch vorhandenes Monoethylamin und Wasser abdestilliert. Die Energiezufuhr kann mit einem Umlaufverdampfer erfolgen. Das Destillat ist eine bevorzugt 15 bis 30 Gew.-%ige, besonders 15 bis 25 Gew.%ige, z.B. ca. 20 Gew.-%ige, wässrige Monoethylaminlösung. Aus der Lösung kann mit bekannten Verfahren wasserfreies Monoethylamin zurückgewonnen werden.

Bevorzugt erfolgt die Abdestillation des noch vorhandenen Monoethylamins und des Wassers in dieser Destillationsstufe in Gegenwart von 0,05 bis 1 Gew.-%, besonders 0,05 bis 0,2 Gew.-%, ganz besonders 0,08 bis 0,15 Gew.-%, (jeweils bezogen auf die Menge an NEP im Zulauf dieser Destillationsstufe) Hydroxiden der Metalle Na, K, Li, Ba oder Ca.

Besonders bevorzugt erfolgt die Abdestillation des MEA/Wasser-Gemischs in dieser Destillationsstufe in Gegenwart von NaOH, das als Natronlauge mittels einer Pumpe in den Zulauf der Kolonne K1 dosiert wird. Z.B. wird hier wässrige 25 %ige Natronlauge so eingesetzt, dass die Konzentration an NaOH 0,05 bis 0,2 Gew.%, insbesondere 0,08 bis 0,15 Gew.-%, (jeweils bezogen auf die Menge an NEP im Zulauf dieser Destillationsstufe) beträgt.

Erfindungsgemäß wurde erkannt, dass durch diese besondere Verfahrensausgestaltung im Reaktionsprodukt enthaltene saure Komponenten (welche Korrosionsprobleme in den Apparaten bereiten können) und ggf. restliche Spuren an unumgesetzten GBL gebunden werden (Umsetzung des GBLs zum entsprechenden Metallsalz der gamma-Hydroxybuttersäure), so dass schließlich ein besonders reines NEP erhalten wird.

Anschließend wird in einer bevorzugten Fahrweise der Sumpf aus der Kolonne K1 entnommen, gegebenenfalls abgekühlt und mit einer Pumpe zur Kolonne K2 zwecks Reindestillation des NEPs befördert. In der Kolonne K2 wird reines NEP als flüssiger Seitenabzug gewonnen. Die Sumpftemperaturen betragen in der Regel 100 bis 140°C, bei 0,01 bis 0,02 bar. (Siedepunkt von NEP: 212°C / 1,013 bar). Die Wärmezufuhr kann auch hier mit einem Umlaufverdampfer erfolgen. Das Kopfdestillat, das neben NEP auch MEA und Wasser enthält, kann zur Kolonne K1 (siehe oben) und/oder zum Reaktoreingang zurückgeführt werden.

Das Kopfdestillat der Kolonne K1 wird bevorzugt einer Kolonne K3 zugeführt, in der das im Destillat enthaltene Monoethylamin für den erneuten Einsatz in der Synthese zurückgewonnen wird.
In der Kolonne K3 erfolgt bevorzugt auch die Entwässerung der MonoethylaminLösung, wonach das erhaltene MEA als frisches Edukt in den Prozess gelangt.

Nach dem erfindungsgemäßen Verfahren wird NEP nach Destillation in Ausbeuten > 96 %, insbesondere ≥ 97,5 %, ganz besonders ≥ 98 % erhalten.

Der Umsatz an GBL beträgt bevorzugt > 99 %, insbesondere ≥ 99,5 %, ganz besonders ≥ 99,9 %.

Die Selektivität (bezogen auf GBL) beträgt bevorzugt > 96 %, z.B. > 97 %, insbesondere ≥ 98 %, ganz besonders ≥ 99 %.

Das erfindungsgemäß erhaltene NEP besitzt nach Destillation eine hohe Qualität:

Die Reinheit beträgt bevorzugt > 99 Gew.-%, insbesondere ≥ 99,5 Gew.-%, ganz besonders ≥ 99,8 Gew.-%.

Der Gehalt an GBL beträgt bevorzugt ≤ 0,05 Gew.-%, insbesondere ≤ 0,02 Gew.-%, z.B. 0 bis 0,01 Gew.-%,
der Gehalt an Monoethylamin beträgt bevorzugt ≤ 1000 ppm, weiter bevorzugt ≤ 800 ppm, besonders ≤ 100 ppm, weiter besonders ≤ 50 ppm, insbesondere ≤ 20 ppm, z.B. 0 bis 15 ppm; (ppm-Angaben jeweils bezogen auf Gewichtsteile).

Die APHA-Farbzahl nach DIN ISO 6271 des erfindungsgemäß erhaltenen NEPs beträgt bevorzugt ≤ 20, insbesondere ≤ 10, und liegt z.B. im Besonderen im Bereich von 2 bis 8.

Das erfindungsgemäße Verfahren kann auch in einer Apparatur (Rohrreaktor), wie sie in der Schrift DE-A-17 95 007, auf die hier diesbezüglich ausdrücklich Bezug genommen wird, beschrieben ist, ausgeführt werden.

Das erfindungsgemäße Verfahren wird bevorzugt einstufig, d.h. in einem Reaktor, der auch aus apparatetechnischen Gründen in zwei oder mehrere Apparate (Reaktoren) aufgeteilt sein kann, wobei dann in jedem dieser Reaktoren die erfindungsgemäßen Bedingungen bezüglich Druck und Temperatur herrschen, durchgeführt.

In einer alternativen Ausgestaltung des erfindungsgemäßen Verfahrens können auch mehrere Reaktoren, insbesondere Rohrreaktoren, (z.B. zwei oder drei Reaktoren, jeweils wie oben beschrieben) in Sumpffahrweise hintereinander geschaltet werden, wobei mindestens in einem dieser Reaktoren, bevorzugt im letzten dieser Reaktoren, die erfindungsgemäßen Reaktionsbedingungen bezüglich Druck und Temperatur herrschen.

Ein Beispiel für eine solche Reihenschaltung von mehreren Reaktoren findet sich in der Schrift WO-A-99/52867 (siehe dort das Verfahrensschema, Reaktoren Nr. 5, 9 und 13 und die Beschreibung), auf die hier diesbezüglich ausdrücklich Bezug genommen wird.

### Beispiele

Die NEP-Syrithese wurde unter den unten genannten Bedingungen in kontinuierlicher Fahrweise in einer Produktionsanlage durchgeführt, die weitgehend dem oben dargestellten Verfahrenskonzept entsprach, d.h. die Edukte GBL und MEA wurden in einem rekuperativen Wärmetauscher sowie zwei dampfbeheizten Wärmetauschern vorgewärmt und der Synthese zugeführt. In einer der Synthese nachgeschalteten Kolonne wurde das überschüssige Ethylamin sowie das dem Prozess zugeführte Wasser abgetrennt und aus dem Prozess ausgeschleußt. In einer zweiten Kolonne erfolgte die Abtrennung des Rückstandes und die Reindestillation des Produktes. Auf die Aufbereitung und Wiederverwertung des in der ersten Kolonne abgetrennten Ethylamins wurde verzichtet.

Der Reaktor wurde in Sumpffahrweise im geraden Durchgang betrieben. Die Einsatzstoffe Monoethylamin (MEA) und GBL wurden vorgeheizt und mittels Pumpen zum Reaktoreingang gefördert, wo die Mischung der beiden Ströme erfolgte. Der Gesamtwassergehalt der Einsatzstoffe lag bei 14 Gew.-%.

Die Reaktion wurde während des Betriebsversuches bei folgenden Bedingungen durchgeführt:

| Versuch Nr. 1: | |
|---|---|
| Temperatur: | 347 - 355°C |
| Druck: | 90 bar |
| Belastung: | 0,6 kg GBL / (Reaktor h) |
| MEA : GBL - Molverhältnis | 1,04 |

| Versuch Nr. 2: | |
|---|---|
| Temperatur: | 353 - 366°C |
| Druck: | 96 bar |
| Belastung: | 1,2 kg GBL / (I_{Reaktor} • h) |
| MEA : GBL - Molverhältnis | 1,14 |

| | |
|---|---|
| Reaktorbelastung in kg GBL pro Liter Reaktorvolumen und Stunde. | |

In der folgenden Tabelle ist die Zusammensetzung des Produktstroms aufgeführt, der in der Produktionsanlage gewonnen wurde.

**Tabelle: GC-Analyse**

| Versuch Nr. | NEP [GC-FI.%] | GBL [GC-FI.%] | M-NEP*) [GC-FI.%] | Sonstige [GC-FI.%] |
|---|---|---|---|---|
| 1 | 99,85 | 0,038 | 0,10 | 0,01 |
| 2 | 99,82 | 0,034 | 0,11 | 0,03 |

| | | | | |
|---|---|---|---|---|
| GC-Bedingungen: 30 m DB-1, Temperaturprogramm: 80°C Eingangstemperatur, 4°C/Min. Aufheizrate, 250°C Endtemperatur. Der H₂O-Restgeha ist nicht berücksichtigt. ^{*}) M-NEP = Methyl-N-Ethyl-2-Pyrrolidon | | | | |

Die NEP-Ausbeute im Rohaustrag in Bezug auf das eingesetzte GBL betrug > 99 %.

Die Raum-Zeit-Ausbeute an NEP im Reaktorrohaustrag lag bei 0,64 kg NEP / (h • I_{Reaktor}) (Versuch 1) bzw. 1,35 kg NEP / (h • I_{Reaktor}) (Versuch 2).

## Patentansprüche

1. Verfahren zur kontinuierlichen Herstellung von N-Ethyl-2-pyrralidon (NEP) durch Umsetzung von gamma-Butyrolacton (GBL) mit Monoethylamin (MEA) in der Flüssigphase bei einer Temperatur von 320 bis 420°C und einem Absolutdruck von 70 bis 120 bar, wobei das Molverhältnis der Einsatzstoffe GBL : MEA im Bereich von 1 : 1,08 bis 1 : 2 liegt und das Einsatzstoffgemisch am Reaktoreingang weniger als 15 Gew.-% Wasser enthält und man das erhaltende Reaktionsprodukt als Zulauf einer Kolonne K1 zuführt, in der MEA und Wasser abdestilliert wird, **dadurch gekennzeichnet, dass** man das Kopfdestillat der Kolonne K1 einer weiteren Kolonne K3 zuführt, in der die Entwässerung von MEA erfolgt, und dass der Einsatzstoff MEA als wässrige MEA-Lösung in das Verfahren eingesetzt wird, wobei die Entwässerung der wässrigen MEA-Lösung in der Kolonne K3 erfolgt, wonach das erhaltene MEA als Einsatzstoff in den Prozess gelangt.

2. Verfahren nach Anspruch 1, **dadurch gekennzeichnet, dass** man die Umsetzung bei einer Temperatur im Bereich von 340 bis 410°C durchführt.

3. Verfahren nach einem der vorhergehenden Ansprüche, **dadurch gekennzeichnet, dass** man die Umsetzung bei einem Absolutdruck im Bereich von 80 bis 110 bar durchführt.

4. Verfahren nach einem der vorhergehenden Ansprüche, **dadurch gekennzeichnet, dass** man GBL und MEA in einem Molverhältnis im Bereich von 1 : 1,08 bis 1 : 1,5 einsetzt.

5. Verfahren nach einem der vorhergehenden Ansprüche, **dadurch gekennzeichnet, dass** man die Umsetzung einstufig durchführt.

6. Verfahren nach einem der vorhergehenden Ansprüche, **dadurch gekennzeichnet, dass** man die Umsetzung in einem aufrecht stehenden Rohrreaktor durchführt.

7. Verfahren nach dem vorhergehenden Anspruch, **dadurch gekennzeichnet, dass** man die Umsetzung in Sumpffahrweise durchführt.

8. Verfahren nach einem der beiden vorgehenden Ansprüche, **dadurch gekennzeichnet, dass** man das Monoethylamin und das GBL dem Rohrreaktor im Reaktorboden durch einen Zweistoff-Injektor separat zuführt.

9. Verfahren nach einem der vorhergehenden Ansprüche, **dadurch gekennzeichnet, dass** man die Umsetzung in Abwesenheit eines Katalysators durchführt.

10. Verfahren nach einem der vorhergehenden Ansprüche, **dadurch gekennzeichnet, dass** das MEA in einer Reinheit (berechnet wasserfrei) von ≥ 90 Gew.-% eingesetzt wird.

11. Verfahren nach einem der vorhergehenden Ansprüche, **dadurch gekennzeichnet, dass** man nach der Umsetzung aus dem Reaktionsprodukt zunächst Wasser und Monoethylamin (MEA) und schließlich das N-Ethyl-2-pyrrolidon abdestilliert.

12. Verfahren nach dem vorhergehenden Anspruch, **dadurch gekennzeichnet, dass** man das abdestillierte MEA in die Umsetzung mit GBL zurückführt.

13. Verfahren nach einem der vorhergehenden Ansprüche, **dadurch gekennzeichnet, dass** man nach der Umsetzung aus dem Reaktionsprodukt das Wasser und Monoethylamin in Gegenwart von Hydroxiden der Metalle Na, K, Li, Ba oder Ca, insbesondere in Gegenwart von NaOH, abdestilliert.

14. Verfahren nach einem der vorhergehenden Ansprüche, **dadurch gekennzeichnet, dass** man das N-Ethyl-2-pyrrolidon in einer Destillationskolonne als flüssigen Seitenabzug gewinnt.

15. Verfahren nach einem der vorhergehenden Ansprüche zur Herstellung von N-Ethyl-2-pyrrolidon mit einer Selektivität von > 97 %.

16. Verfahren nach einem der vorhergehenden Ansprüche zur Herstellung von N-Ethyl-2-pyrrolidon mit einer Reinheit von ≥ 99,5 Gew.-%.

17. Verfahren nach einem der vorhergehenden Ansprüche zur Herstellung von N-Ethyl-2-pyrrolidon mit einer APHA-Farbzahl von ≤ 20.

18. Verfahren nach einem der vorhergehenden Ansprüche zur Herstellung von N-Ethyl-2-pyrrolidon mit einer Raum-Zeit-Ausbeute von ≥ 1 kg NEP / (h • I_{Reaktor}).

## Claims

1. A process for the continuous preparation of N-ethyl-2-pyrrolidone (NEPs) by reacting gamma-butyrolactone (GBL) with monoethylamine (MEA) in the liquid phase at a temperature from 320 to 420°C and an absolute pressure from 70 to 120 bar with the molar ratio of the starting materials GBL:MEA being in the range from 1:1.08 to 1:2 and the feed mixture at the reactor inlet comprising less than 15% by weight of water and the reaction product obtained being introduced as feed into a column K1 in which MEA and water are distilled off, wherein the overhead distillate from the column K1 is fed to a further column K3 in which the dewatering of MEA takers place and the starting material MEA is introduced as aqueous MEA solution into the process, with the dewatering of the aqueous MEA solution occurring in the column K3 and the resulting MEA then going as starting material into the prowess.

2. The process according to claim 1, wherein the reaction is carried out at a temperature in the range from 340 to 410°C.

3. The process according to either of the preceding claims, wherein the reaction is carried out at an absolute pressure in the range from 80 to 10 bar.

4. The process according to any of the preceding claims, wherein GBL and MEA are used in a molar ratio in the range from 1:1.08 to 1:1.5.

5. The process according to any of the preceding claims, wherein the reaction is carried out in a single stage.

6. The process according to any of the preceding claims, wherein the reaction is carried out in an upright tube reactor.

7. The process according to the preceding claim, wherein the reaction is carried out in the upflow mode,

8. The process according to either of the two preceding claim, wherein the monoethylamine and the GBL are fed separately into the tube reactor at the bottom of the reactor via a two-fluid injector.

9. The process according to any of the preceding claims, wherein the reaction is carried out in the absence of a catalyst.

10. The process according to any of the preceding claims, wherein the MEA is used in a purity (calculated on a water-free basis) of ≥ 90% by weight.

11. The process according to any of the preceding claim, wherein firstly water and monoethylamine (MEA) and finally the N-ethyl-2ypyrrolidone are distilled off from the reaction product after the redaction.

12. The process according to the preceding claim, wherein the MEA which has been distilled off is recirculated to the reaction with GEL.

13. The process according to any of the preceding claims, wherein the water and monoethylamine are distilled off from the reaction product in the presence of hydroxides of the metals Na, K, Li, Ba or Ca, in particular in the presence of NaOH, after the reaction.

14. The process according to any of the preceding claims, wherein the N-ethyl-2-pyrrolidone is isolated as a liquid stream from a side offtake of a distillation column.

15. The process according to any of the preceding claims for preparing N-ethyl-2. pyrrolidone with a selectivity of > 97%.

16. The process according to any of the preceding claims for preparing N-ethyl-2-pyrrolidone having a purity of ≥ 99.5% by weight.

17. The process according to any of the preceding claims for preparing N-ethyl-2-pyrrolidone having an ALPHA color number of ≤ 20.

18. The process according to any of the preceding claims for preparing N-ethyl-2-pyrrolidone in a space-time yield of ≥ 1 kg NEP/(h • I_{reactor}).

## Revendications

1. Procédé de fabrication continue de N-éthyl-2-pyrrolidone (NEP) par réaction de gamma-butyrolactone (GBL) avec de la monoéthylamine (MEA) en phase liquide à une température de 320 à 420°C et une pression absolue de 70 à 120 bars, le rapport molaire entre les substances de départ GBL:MEA se situant dans la plage allant de 1:1, 08 à 1:2, le mélange des substances de départ contenant moins de 15 % en poids d'eau à l'entrée du réacteur et le produit de réaction obtenu étant introduit en tant qu'alimentation dans une colonne K1, dans laquelle la MEA et l'eau sont séparées par distillation, **caractérisé en ce que** le distillat de tête de la colonne K1 est introduit dans une colonne K3 supplémentaire, dans laquelle la déshydratation de la MEA a lieu, et **en ce que** la substance de départ MEA est utilisée dans le procédé sous la forme d'une solution aqueuse de MEA, la déshydratation de la solution aqueuse de MEA ayant lieu dans la colonne K3 et la MEA obtenue étant ensuite utilisée dans le procédé en tant que substance de départ,

2. Procédé selon la revendication 1, **caractérisé en ce que** la réaction est réalisée à une température dans la plage allant de 340 à 410°C.

3. Procédé selon l'une quelconque des revendications précédentes, **caractérisé en ce que** la réaction est réalisée à une pression absolue dans la plage allant de 80 à 110 bars.

4. Procédé selon l'une quelconque des revendications précédentes, **caractérisé en ce que** la GBL et la MEA sont utilisées en un rapport molaire dans la plage allant due 1:1, 08 à 1:1,5.

5. Procédé selon l'une quelconque des revendications précédentes, **caractérisé en ce que** la réaction est réalisée en une étape.

6. Procédé selon zone quelconque des revendications précédentes, **caractérisé en ce que** la réaction est réalisée dans un réacteur tubulaire vertical.

7. Procédé selon la revendication précédente, **caractérisé en ce que** la réaction est réalisée en mode de fond.

8. Procédé selon l'une quelconque des deux revendications précédentes, **caractérisé en ce que** la monoethylamine et la GBL sont introduites séparément dans le réacteur tubulaire dans le fond du réacteur par un injecteur à deux substances.

9. Procédé selon l'une quelconque des revendications précédentes, **caractérisé en ce que** la réaction est réalisée en l'absence d'un catalyseur.

10. Procédé selon l'une quelconque des revendications précédentes, **caractérisé en ce que** la MEA est utilisée en une pureté (calculée anhydre) ≥90% en poids.

11. Procédé selon l'une quelconque des revendications précédentes, **caractérisé en ce que** tout d'abord l'eau et la monoéthylamine (MEA), puis la N-éthyl-2-pyrrolidone, sont séparées par distillation du produit de réaction après la réaction.

12. Procédé selon la revendication précédente, **caractérisé en ce que** la MEA séparée par distillation est recyclée dans la réaction avec la GBL.

13. Procédé selon l'une quelconque des revendications précédentes, **caractérisé en ce que** l'eau et la monoéthylamine sont séparées par distillation du produit de réaction après la réaction en présence d'hydroxydes des métaux Na, K, Li, Ba ou Ca, notamment en présence de NaOH.

14. Procédé selon l'une quelconque des revendications précédentes, **caractérisé en ce que** la N-éthyl-2-pyrrolidone est obtenue dans une colonne de distillation en tant que sortie latérale liquide.

15. Procédé selon l'une quelconque des revendications précédentes, pour à fabrication de N-éthyl-2-pyrrolidone avec une sélectivité > 97 %.

16. Procédé selon l'une quelconque des revendications précédentes, pour la fabrication de N-éthyl-2-pyrrolidone avec une pureté à 99,5 %.

17. Procédé selon l'une quelconque des revendications précédentes, pour la fabrication de N-éthyl-2-pyrrolidone avec un indice de couleur APHA ≤ 20.

18. Procédé selon l'une quelconque des revendications précédentes, pour la fabrication de N-êthyl-2-pyrrolidone avec un rendement espace-temps ≥ 1 kg NEP/(h·1_{réacteur}).
